# EUROPEAN PATENT SPECIFICATION

(11) **EP 1 636 180 B1**
(45) Date of publication and mention of the grant of the patent: **18.07.2007**
(21) Application number: 04733961.9
(22) Date of filing: 19.05.2004
(51) Int. Cl.: C07D 201/16, C07D 207/267

(54) **PROCESS FOR RECTIFYING N-VINYL-2-PYRROLIDONE**
VERFAHREN ZUR REINIGUNG VON N-VINYLPYRROLIDON
PROCEDE DE RECTIFICATION DE N-VINYL-2-PYRROLIDONE

(30) Priority: 21.05.2003 JP 2003143731
(43) Date of publication of application: 22.03.2006
(73) Proprietor: NIPPON SHOKUBAI CO., LTD., Osaka-shi, Osaka 541-0043 (JP)
(72) Inventor: SUGIURA, Hideto, 2100805 (JP); YANO, Hitoshi, 5620032 (JP)
(74) Representative: Luderschmidt, Schüler & Partner
(86) International application number: PCT/JP2004/007130
(87) International publication number: WO 2004/103965

(56) References cited:
- US-A- 5 329 021
- US-A- 5 710 284
- US-A1- 2003 004 284

## Description

### Technical Field:

This invention relates to a process for obtaining N-vinyl-2-pyrrolidone of high purity (the term "N-vinyl-2-pyrrolidone" may be occasionally referred to as "NVP") by crystallization.

### Background Art:

Polymers of vinyl pyrrolidone are being widely used in the fields of cosmetic preparations, chemicals, pharmaceutical preparations, food additives, personal care articles and the like. In these fields, requirements for high purity products and for removing odor and color forming impurities are strong.

In order to respond to these requirements, processes for removing these impurities from NVP have been proposed (United States Patent Nos. 5710284 and 5329021).

### Disclosure of Invention

No process for improving the degradation of the yield due to the NVP polymerization during the course of crystallization, however, is described anywhere in such patent publications.

An object of this invention, therefore, consists in improving the purity of NVP and remedying the degradation of yield in a process for rectifying NVP by crystallization.

This invention relates to a process for rectifying N-vinyl-2-pyrrolidone by crystallization, characterized in that the crystallization is performed for a mother liquid having a crude N-vinyl-2-pyrrolidone in the presence of a stabilizer.

In accordance with the present process, it can provide a high purity NVP and improve the degradation of yield by a very simple crystallization procedure in the presence of a stabilizer.

The above and other objects, features and advantages of the present invention will become clear from the following

### description of the preferred embodiments.

### Brief Description of Drawings

The accompanying drawing incorporated in and forming a part of the specification, illustrates several aspects of the present invention, and together with the description serve to explain the principles of the invention. In the drawings:
Fig. 1 is a block diagram illustrating a process for rectifying NVP by crystallization contemplated by this invention;
Fig. 2 is a partially cutaway schematic cross section illustrating a multistage cooling and crystallization device used in this invention;
Fig. 3 is a schematic cross section illustrating a column type rectification device used in this invention;
Fig. 4 is a block diagram illustrating a distillation device used for rectifying the raw material liquid; and
Fig. 5 is a block diagram illustrating another example of the distillation device used for rectifying the rawmaterial liquid.

### Best Mode for Carrying Out the Invention

This invention relates to a process for rectifying or purifying NVP by crystallization, characterized in that the crystallization is performed in a mother liquid having NVP as a main component and containing impurities (crude NVP) in the presence of a stabilizer or stabilizing agent. The expression "rectification by crystallization" as used herein refers to a rectifying process in which at least one step of crystallization is included. Examples of the process may include a process of multistage fractional crystallization, a process which comprises a single or a plurality of dynamic and static steps of crystallization, and a process which comprises performing a crystallization treatment and subsequently rectifying the resultant crystals. In the present specification, the crystallization and rectification process will be explained as a typical example.

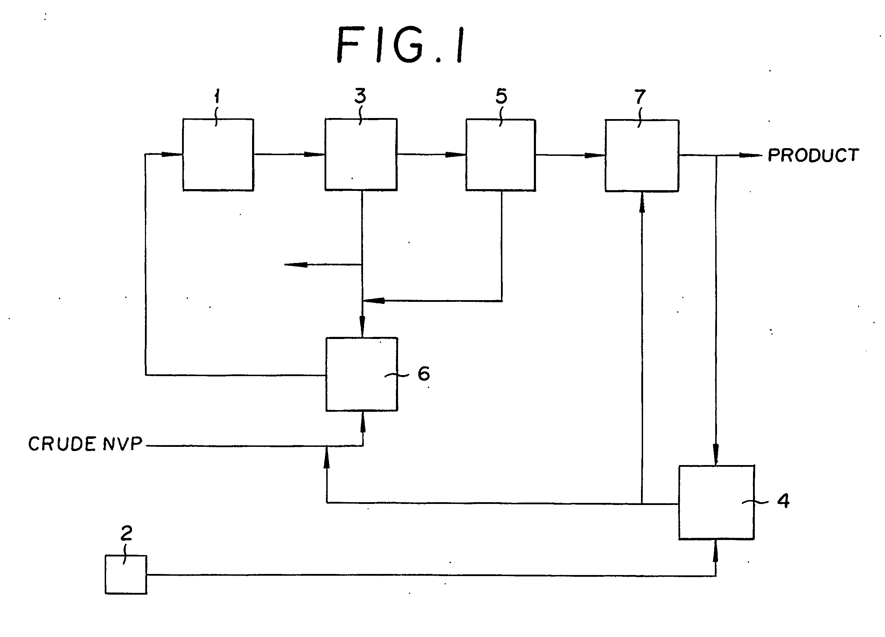

Fig. 1 is a block diagram illustrating a process for rectifying NVP by crystallizing it in this invention. In Fig. 1, a mother liquid which contains crude NVP is made to add a stabilizer 2 and then fed to a crystallization step 1. Incidentally, the stabilizer 2 is dissolved or dispersed in a high concentration in advance in refined NVP (a stabilizer mixture step 4). A slurry which emanates from the crystallization step 1 is filtered at a filtration step 3. Crystals obtained at the filtration step 3 are fed to a crystal rectification step 5. Further, refined NVP at the crystal rectification step 5 is fed to a tank (a refined NVP storage step 7), and made to add the stabilizer 2. The stabilizer 2 at the crystallization step 1 and the storage step 7 may be identical as shown in Fig. 1 or different from each other (not shown). A separated NVP-containing filtrate from the filtration step 3 and the separated NVP-containing liquid from the crystal rectification step 5 are added to the mother liquid by way of reclamation (a recovered liquid mixture step 6).

Now, the raw materials and steps to be used in this rectifying process will be described below in order.

### (i) Raw material

The crude NVP which is refined by this rectifying process is not particularly restricted. Examples of the crude NVPs, which are used for the purpose of this rectification, may include those obtained by the vinylation of 2-pyrrolidone with acetylene; obtained by dehydrochlorination by transforming butyrolactone by the action of ethanolamine into 1-(β-oxyethyl)-2-pyrrolidone and converting the hydroxyl group with thionyl chloride into chloro group; obtained by the removal of acetic acid from the acetic ester intermediate resulting from the reaction of acetic anhydride and N-(2-hydroxyethyl)-2-pyrrolidone; and obtained by the gas phase dehydrating reaction of N-hydroxyethyl-2-pyrrolidone (occasionally referred to as "gas phase dehydrating reaction"). The crude NVP as such may be fed to the subsequent crystallization step. In the case of the crude NVP which contains impurities copiously, it is preferable to use the NVP after purification by a known process such as fractional distillation, in order that the yield of rectification may be enhanced and the duration of treatment may be shortened as well. In this specification, the crude NVP which has been formed by the gas phase dehydrating reaction and subsequent distillation is described as a typical example.

The gas phase dehydrating reaction will be described. Crude NVP is obtained by subjecting N-hydroxyethyl-2-pyrrolidone to a gas phase intramolecular dehydrating reaction in the presence of a catalyst and purifying the reaction product by distillation. The crude NVP contains generally about 0.5% - about 0.01% impurities, such as water which is a by-product, 2-pyrrolidone which is the production of decomposition of N-hydroxyethyl-2-pyrrolidone, N-hydroxyethyl-2-pyrrolidone which is an unaltered raw material, and polymers. Certainly, NVP-containing liquids which occur during this rectification step may be used as mixed with the crude NVP. This mixture is performed by a known mixing device.

### (ii) Crystallization step

The crystallization contemplated by this invention is, but not restricted, required to be capable of supercooling liquid NVP and extracting crystals therefrom. As regards the crystallization, it may adopt any of the known processes and devices, for example, continuous crystallizers such as forced circulation type devices, multistage crystallizers, conveying layer type devices, classifying layer type devices, turbulent type mixing and classifying devices, double crystallizers, direct refrigerant contact cooling and crystallizers, and devices resorting to the process for formation of an aggregate; and cooling type crystallizers such as tank type crystallizers, Swenson-Walker crystallizer, Howard crystallizer, and drum flakers. In the present specification, the multistage crystallizer will be cited as a typical example.

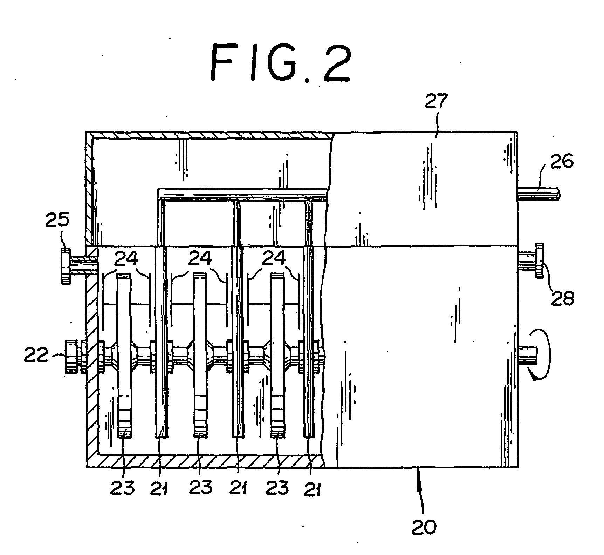

Fig. 2 is a partially cutaway schematic cross section illustrating a multistage cooling type crystallizer, which is used in this invention. In Fig. 2, the device is a horizontal crystallization tank 20 whose interior is partitioned with a plurality of cooling plates 21. The crystallization tank 20 is covered with a thermal insulating material (not shown) and provided with a lid 27. An agitating shaft 22 which is laid through the centers of the cooling plates 21 has disklike agitating vanes 23. The agitating vanes 23 are severally provided with wipers 24 to keep the surfaces of the cooling plates 21 in a clean state. The agitating vanes 23 are rotated at a prescribed speed by a motor (not shown). The raw material liquid enters through a raw material inlet 25 and sequentially moves to the right from the conduit beneath the cooling plates 21. The liquid in the interior moves substantially in the form of a plug flow. The liquid fed overflows from an outlet 28. Though any mode of flow may be adopted for the supply of refrigerants to a plurality of the cooling plates 21, the refrigerant having the same temperature can be delivered parallelly to all the cooling plates 21, for example. The refrigerant is admitted through a refrigerant inlet 26 and withdrawn via an outlet (not shown). By allowing the refrigerant to flow in this manner, it is possible to prevent the occurrence of local supercooling and excessive formation of nuclei in the crystallizer.

In Fig. 2, the NVP in the mother liquid is crystallized by utilizing a refrigerant such as water or an aqueous ethylene glycol solution. Here, the fused or dissolved NVP-containing raw material for the crystallization is referred to as "mother liquid." The crystallization is performed by adjusting the temperature of the withdrawn liquid (in the range of -20°C to 13. 5°C, for example) so as to keep the slurry concentration in the mother liquid at a prescribed level for example in the range of 5 - 60%, and treating the mother liquid for a duration of about four hours.

In this case, it is important that the crystallization in the mother liquid should proceed in the presence of a stabilizer. The stabilizer is, but not restricted, required to be capable of preventing the NVP and the impurities contained therein from being polymerized, for example, a phenol type oxidation inhibitor or an amine type polymerization inhibitor. Examples of the phenol type oxidation inhibitors may include 2,6-di-t-butyl-4-hydroxytoluene, 2,6-di-t-butyl-p-cresol, butylated hydroxyanisole, stearyl β-(3,5-di-t-butyl-4-hydroxyphenyl) propionate, 2,2'-methylene-bis(4-methyl-6-t-butylphenol), 4,4'-thiobis(3-methyl-6-t-butylphenol), 4,4'-butylidene-bis(3-methyl-6-t-butylphenol), 1,1,3-tris(2-methyl-4-hydroxy-5-t-butylphenyl)butane, 1,3,5-trimethyl-2,4,6-tris(3,5-di-t-butyl-4-hydroxybenzy 1) benzene, and tetrakis[methylene-3-(3',5'-di-t-butyl-4'-hydroxyphenyl) propionate]methane. Examples of the amine type polymerization inhibitors may include diallyl amines such as ketone-amine condensates, diallyl diamine, N,N-dimethylbenzylamine, N,N-dimethylaniline, P-N, N-dimethylaminopiperazine; ketone-diallylamine condensates, triethylamine, tri-n-butylamine, N-methylmorphine, and phenylenediamine. Naturally, these stabilizers may be used singly or in a combination thereof. Among them, the phenylenediamine type polymerization inhibitor or the phenol type oxidation inhibitor proves particularly advantageous from the viewpoint of the ability to prevent the NVP from being polymerized. Particularly, N,N'-di-sec-butyl-p-phenylenediamine, p-phenylenediamine, N,N'-diethyl-p-phenylenediamine, N,N'-diphenyl-p-phenylenediamine and 2,6-di-t-butyl-4-hydroxytoluene prove favorable. The recovered liquids obtained from the filtration and crystal rectification steps, hereinafter described, are put to use again as such when they contain the stabilizer in the prescribed amount. When no stabilizer is included, it is added to the liquid prior to the crystallization. The amount of the stabilizer is, but not restricted, required to be enough for precluding or allaying the polymerization of the contents such as NVP in the crystallization step, generally not exceeding 1.5% by weight (not inclusive of 0) and preferably falling in the range of 0.1 ppm - 1.0% by weight, based on the weight of the NVP.

The stabilizer may be added as such to the raw material liquid or the mother liquid. In the case of low concentration of the stabilizer, the following is preferable that the stabilizer is dissolved or dispersed in advance in a prescribed concentration, e.g. 0.1 - 10 g/kg in the refined NVP, raw material liquid, or mother liquid and then the resultant medium is added to the liquid. The mixing is implemented using a known agitating device. By using the medium of this nature, it is possible to have the stabilizer added accurately and quickly and thoroughly dispersed or dissolved.

By performing the crystallization in the presence of the stabilizer at the prescribed amount, it is possible to preclude or allay the polymerization of the NVP and prevent the viscosity of the liquid or slurry from increasing. It is inferred consequently that the amount of the impurities suffered to adhere to NVP crystals can be decreased during the filtration of the NVP crystals and the increase of purity can be promoted during the crystal rectification step.

The slurry which is withdrawn via the outlet is fed to the subsequent filtration step.

### (iii) Filtration step

The filtration is, but not restricted, required to be capable of separating crystal phase obtained and residual liquid phase. Any of the known continuous and batchwise processes of filtration can be adopted. It is preferable to use a belt type filter from the viewpoint of ease of the shift from the crystallization step to the crystal rectification step.

Since the filtrate obtained from the filtration step contains generally not less than 95% of NVP, though variable with the conditions of crystallization, it is fed to the crystallization step again for recovering NVP. The filtrate generally contains the stabilizer, and does not require new addition of the stabilizer. When the stabilizer in an amount is smaller than the prescribed, the filtrate is fed to the crystallization step after addition of the stabilizer.

When the separated liquid from the filtration step and the separated liquid from the crystal rectification step, which will be describedbelow, are reclaimed as the rawmaterial for crystallization, the impurities in the mother liquid are concentrated with the precipitation of crystals. The refined NVP obtained at a higher impurity concentration has the possibility of deficiency in quality. It is, therefore, suitable to control the concentration of impurities in the mother liquid without affecting adverse effects to the quality of refined NVP.

The control of the impurity concentration in the mother liquid at the crystallization step is advantageously accomplished at the time that the concentration reaches a level requiring the control, for example, by supplying a virgin crude NVP to the crystallization step so as to reduce or control the concentration of impurities within the system of the crystallization step below a fixed level (not inclusive of 0) such as below 10% and preferably below 6%, while removing part or the whole of the filtrate obtained from the filtration step out of the system without being supplied.

It is proper to forward the filtrate to the crystallization step while removing the filtrate in a prescribed amount, about 3%, based on the weight of the crude NVP, from the rectification systemnot to increase the impurity concentration in the mother liquid. This value of 3% is a magnitude which depends on the purity set as the target and, therefore, is required to be increased in accordance as the target purity increases. This is because there is a tendency that the higher you want to rectify NVP, the more preferable the purity of NVP in the crude NVP is higher. Incidentally, the removed NVP-containing filtrate from the rectification system may be reclaimed by re-distillation or crystallization to the same degree of purity, for example.

The crude crystals or cakes of NVP obtained from the filtration step are fed to the subsequent crystal rectification step. Incidentally, the term "NVP crystals" are adopted in referring to the crystals of NVP in the general sense even embracing crude crystals of NVP, whereas the term "crude crystals of NVP" is used in discriminating the crystals that contain impurities before rectification.

### (iv) Crystal rectification step

The term "crystal rectification" as used herein refers to the rectification of crystals by utilizing the phenomenon of sweating. The sweating is, but not restricted, required to be capable of fusing part of NVP crystals, in other words fusing a comparatively impure part of the crystals, thereby enabling the crystals to be refined. For the operation of the sweating itself, known devices and processes are available such as a falling-film type device (MWB) and column type rectification device. The MWB repeats the procedure of causing a raw material to flow down the inside of a cylinder in the formof a liquid film, inducing precipitation of crystals on the cylinder by means of a refrigerant, allowing the precipitate to grow to a prescribed thickness, then elevating the temperature of the refrigerant thereby inducing sweating and allowing impurities to flow down, and thereafter fusing the remaining layer of crystals. The column type rectification device performs the rectification of crystals by transferring the crystals upward and meanwhile allowing them to contact a reflux liquid and consequently inducing sweating.

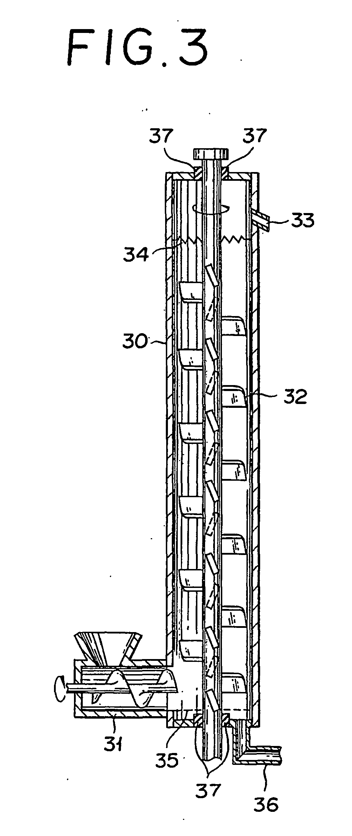

Fig. 3 is a schematic cross section illustrating a column type rectification device to be used in this invention. In Fig. 3, a feeder 31 is disposed at the lower part of a column 30 and two agitation shafts 32 equipped with several vanes are disposed inside the column 30. This device, as viewed from above the column 30, appears to be in a biaxial structure which resembles eyeglasses. In Fig. 3, only one of the two shafts is shown. The two agitation shaft 32 equipped with several vanes are rotated at a prescribed speed, e.g. 1 - 2 min⁻¹ using a motor (not shown). The column is provided at the upper part thereof with a product output port 33. A heating means 34 (indicates only the place set. not shown) is disposed below the product outlet port 33 and in the upper part of the column 30 to fuse part of the cake. The column 30 is provided near the bottom part thereof with a perforated plate 35 which is underlain by a fused liquid outlet port 36. At the top part and the bottom part of the column 30, seals 37 are formedbetweenthe two agitation shafts 32 provided with vanes and the column 30. Incidentally, the main body part of the column 30 is covered with a thermal insulating material (not shown).

In Fig. 3, the cake obtained from the filtration step is continuously supplied by the feeder 31 which is disposed at the lower part of the column 30. The cake is transported upward by the two agitation shafts 32 provided with vanes which are rotated at a low speed in opposite directions. Most of the cake which has reached the upper part of the column 30 is withdrawn from the system via the product outlet port 33, and the remainder thereof fused by the heater 34 is allowed to flow as a reflux liquid down the interior of the column 30 by the gravitational attraction. This reflux liquid, by contacting the cake, is enabled to fuse part of the cake and induce the sweating phenomenon for transferring the impurities in the cake to the surface thereof. Since these impurities are captured by the descending reflux liquid, the reflux liquid has the impurity concentration increased in proportion toward the lower part of the column 30, and eventually the concentrated impurities are withdrawn from the lower part of the column 30. The device is so constructed that the impurity-containing liquid may be passed through the perforated plate 35 and withdrawn from the fused liquid outlet port 36 and the cake may not easily fall down.

The ratio of the NVP crystals to be fused for the sake of reflux is, but not restricted, required to fall generally in the range of 5 - 70% by weight and preferably in the range of 10 - 50% by weight, based on the weight of the cake obtained.

The agitation shafts serve to enhance the efficiency of solid-liquid contact. Particularly where the vanes of the two shafts overlap, the action of sweating is promoted because the cake is squeezed.

The crystal rectification step operates by partially fusing the cake mentioned above with hot water having a temperature of 20°C - 50°C. The retention time is generally in the range of 0.5 - 3 hours. The liquid emanated from the fused liquid outlet port 36 contains about 99% NVP, though variable with the rectifying conditions. It is therefore mixed with the filtrate mentioned above and fed as a recovered liquid to the crystallization step. The liquid which emanates from the fused liquid outlet port 36 still entrains the stabilizer.

Meanwhile, the cake which has been treated at the crystal rectification step is fused to be fed to a tank.

It is inferred that at the crystal rectification step, the impurities are rectified by the following reason. The crude crystals of NVP do not necessarily have a high purity because the crystals are contaminated with the mother liquid, e.g., adhered thereto or incorporated therein, while the crystals themselves have a high purity. Thus, the rectification is performed by the sweating operation which serves to retain the crystals at a rather higher temperature than the crystallization temperature. When the crude crystals of NVP are washed with the fused liquid descending from the top of the column, the mother liquid adhering to their surfaces are removed. When the crystals are partially fused, the mother liquid incorporated in the crystals is fed through the resultant conduit to the surface of the crystals and the impurities will be selectively removed.

Further, the rectification which is performed by the conventional distillation possibly fails to perform substantial removal of the impurities, depending on the boiling point thereof contained to the NVP. In contrast, the aforementioned "sweating phenomenon" utilized in the process of this invention is based on the partial fusion of NVP and is exhibited in spite of undulations of the melting point of the contained impurities. By the process of this invention, therefore, the contained impurities can be removed from the crude crystals of NVP by the utilization of the aforementioned sweating phenomenon despite of undulations of the melting point thereof.

### (v) Storage step

The refined NVP which has been introduced into the tank contains no stabilizer. It is, therefore, made to add the stabilizer in a prescribed amount, e.g. not more than 1.5% by weight and preferably not more than 1% by weight (not inclusive of 0), based on the weight of the NVP. This stabilizer may be the same as or different from the stabilizer used at the crystallization step. The stabilizer maybe added directly in the refined NVP. Where the concentration of the stabilizer is low, it is proper to dissolve or disperse the stabilizer in a refined NVP at a prescribed concentration for example 0.1 - 10 g/kg and then the resultant medium containing the stabilizer is added. By this addition, it is possible to perform the addition accurately and quickly and induce thorough dispersion or dissolution.

The purity of the refined NVP is generally in the range of about 99.9 - 100% and preferably in the range of about 99.996 - 100%. When the filtrate emanating from the filter is subj ected to the crystal rectification without the addition of any stabilizer, the ratio of loss is 3% relative to the crude NVP. When the crystal rectification is performed in the presence of a stabilizer, however, the ratio of loss can be decreased by several % - 50% and preferably about 50%.

As a preferred embodiment of this invention, it may be cited a process which comprises, in the presence of a stabilizer, implementing the crystal rectification step for precipitating crystals in a mother liquid by way of crystallization and rectifying the crystals and returning at least part of the recovered liquid arising from the filtration step and the crystal rectification step to the mother liquid for the purpose of reclamation. This process is preferable because it can improve not only the purity, but also the yield, of the produced NVP.

### EXAMPLES

Now, this invention will be described in detail below with reference to examples. It is provided, however, that the following examples will not limit the present invention and may be executed as modified without departure from the spirit of the invention described hereinabove and hereinafter. All such modifications are embraced within the technical scope of this invention. The denominations "%" and "ppm" as used in the examples are based on weight unless otherwise specified.

The NVP as a raw material and the NVPs in the individual examples are assayed for composition by a gas chromatography for organic substance and the Karl Fischer' s method for water. In the gas chromatography, the assay was performed under conditions permitting determination accurately to 0.5 ppm.

### (Preparation process of NVP)

The NVP as the raw material is obtained by subjecting N-hydroxyethyl-2-pyrrolidone to a reaction of gas phase dehydration and then purifying the product by distillation.

A solution of 3.45 g of lithium nitrate in 50 g of water was heated and stirred at 90°C. The hot stirred solution and 30 g of silicon oxide added thereto heated and concentrated and then dried in air at 120°C for 20 hours. The solid substance consequently obtained was crushed to 9 - 16 meshes and then calcined in air at 500°C for two hours to prepare a catalyst having a composition of Li₁Si₁₀ in atomic ratio excluding oxygen. A reaction tube made of stainless steel and measuring 10 mm in inside diameter was packed with 5 mL of the catalyst obtained and this reaction tube was immersed in a fused salt bath at 400°C.

To the reaction tube, a feed obtained by diluting N-(2-hydroxyethyl)-2-pyrrolidone with nitrogen gas till the partial pressure thereof reached 1.01 x 10⁴ Pa (76 mmHg) was supplied at a space velocity of 200 h⁻¹ to induce a reaction. The resultant reaction gas excepting nitrogen gas was cooled and collected.

The collected liquid was found to contain NVP, the feed, and compounds having higher boiling points than NVP (excluding the feed), water, and an odorous component.

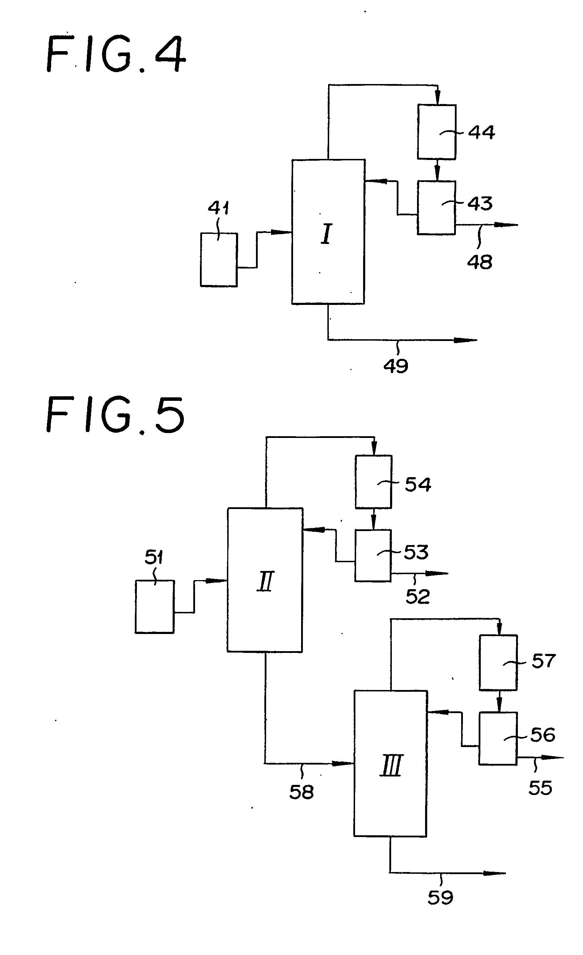

Fig. 4 is a block diagram illustrating a distillation device to be used for rectifying the raw material liquid. Using the device shown in Fig. 4, the collected liquidmentioned above was continuously distilled as the raw material liquid. The part of this distillation device which was exposed to the liquid was made of stainless steel, such as SUS 304, and the gasket was made of Teflon (a polytetrafluoroethylene resin). The raw material liquid from a tank 41 was distilled in a continuous distillation column I (first distillation column) to expel water and the odorous component from the top thereof through a conduit 48 and to obtain a dehydrated liquid as bottoms from the bottom thereof. The continuous distillation column I was made of a glass tube 35 mm, and formed by filling Sulzer packings of stainless steel 35 mm in diameter at the concentrating part thereof with 4 elements and at the recovering part thereof with 6 elements. In the continuous distillation column I, an amount of the distillate refluxed from an emitting tank 43 to the continuous distillation column I was so regulated as to set the operating pressure at 1.33 x 10⁴ Pa (100 mmHg) and the reflux ratio at 1. The amount of the raw material liquid supplied from the tank 41 to the continuous distillation column I per unit time (hereinafter referred to as "supplying speed"), the amount of the distilled liquid expelled from the continuous stilling column I and extracted through the conduit 48 via a condenser 44 and an emitting tank 43 per unit time (hereinafter referred to as "distilling speed"), and the amount of bottoms (dehydrated liquid) extracted from the continuous distillation column I via a conduit 49 (hereinafter referred to as "extracting speed") are shown in Table 1 together with the compositions of the relevant liquids. Further, the temperatures of the top and the bottom respectively of the continuous distillation column I are shown in Table 2.

**TABLE 1**

| | | Composition (g/h) | | | |
|---|---|---|---|---|---|
| | | Water | NVP | HEP | High boiling compound |
| Continuous distillation column I | Supplying speed | 43.2 | 327.6 | 20.8 | 8.4 |
| | Distilling speed | 43.2 | 0 | 0 | 0 |
| | Extracting speed | 0 | 321.8 | 20.8 | 14.2 |

| | | | | | |
|---|---|---|---|---|---|
| In Table; NVP: N-vinyl-2-pyrrolidone HEP: N-(2-hydroxyethyl)-2-pyrrolidone High boiling compound: A compound having a higher boiling point than NVP (excluding HEP). | | | | | |

**TABLE 2**

| | Continuous distillation column I |
|---|---|
| Temperature of the top of column (°C) | 53 |
| Temperature of the bottomof column (°C) | 159 |

Fig. 5 is a block diagram illustrating another distillation device to be used for rectifying the raw material liquid. Using the device shown in Fig. 5, a solution obtained by mixing the resultant bottoms (dehydrated liquid) with water of an amount 0.19 times the weight of the bottoms was continuously supplied from a tank 51 to a continuous distillation column II (the second distillation column). The part of this distillation device which was exposed to the liquid was made of stainless steel, such as SUS 304, and the gasket was made of Teflon (a polytetrafluoroethylene resin) . The continuous distillation column II was made of a glass tube 35 mm, and formed by filling Sulzer packings of stainless steel 35 mm in diameter at the concentrating part thereof with 4 elements and at the recovering part thereof with 6 elements. The extracted liquid from the continuous distillation column II was supplied via a conduit 58 to a continuous distillation column III (the third distillation column). The continuous distillation column III was made of a glass tube 50 mm, and formed by filling Sulzer packings of stainless steel 50 mm in diameter at the concentrating part thereof with 7 elements and at the recovering part thereof with 7 elements. In the continuous distillation column II, the amount of the distillate refluxed from an emitting tank 53 to the continuous distillation column II was so regulated as to set the operating pressure at 1.33 x 10⁴ Pa (100 mmHg) and the reflux ratio at 1. In the continuous distillation column III, the amount of the distillate refluxed from an emitting tank 56 to the continuous distillation column III was so regulated as to set the operating pressure at 1.33 x 10³ Pa (10 mmHg) and the reflux ratio at 1. The reference numerals 54 and 57 each denote a condenser and the reference numerals 52, 55, and 59 each denote a discharge conduit . The supplying speeds, the distilling speeds, and the extracting speeds, respectively, of the continuous distillation columns II and III are shown in Table 3 together with the compositions of the relevant liquids. The temperatures of the top and the bottom, respectively, of the continuous distillation columns II and III are shown in Table 4.

**TABLE 3**

| | | Composition (g/h) | | | |
|---|---|---|---|---|---|
| | | Water | NVP | HEP | High boiling compound |
| Continuous distillation column II | Supplying speed | 68.5 | 321.8 | 20.8 | 14.2 |
| | Distilling speed | 68.5 | 0 | 0 | 0 |
| | Extracting speed | 0 | 318.6 | 20.8 | 17.4 |
| Continuous distillation column III | Supplying speed | 0 | 318.6 | 20.8 | 17.4 |
| | Distilling speed | 0 | 312.2 | 0 | 0 |
| | Extracting speed | 0 | 0 | 20.8 | 23.8 |

**TABLE 4**

| Temperature (°C) | Continuous distillation column II | Continuous distillation column III |
|---|---|---|
| Column top | 53°C | 91°C |
| Column bottom | 159°C | 193°C |

The crude NVP thus obtained by distillation was found to contain 99.92% of NVP, 600 ppm of organic impurities, and 200 ppm of water.

### EXAMPLE 1: CDC + KCP

A raw material NVP (mother liquid) was continuously supplied to a horizontal multistage cooling and crystallization device (CDC, available from GOUDA Corp.) to produce a mother liquid containing crude crystals of NVP. The part of CDC exposed to the liquid and the heat-transmitting cooling plate were made of stainless steel, such as SUS 304, and the wiper, gasket, and shaft seal were made of Teflon (a polytetrafluoroethylene resin). The CDC was operated with the supplying speed of the mother liquid to the CDC set at 75 kg/h, including 10.8 kg/h of the virgin NVP, the slurry concentration set at 20%, the temperature set at 6.5°C, and the retention time at 4 hours. The formation speed for the crude crystals of NVP in this CDC was 15 kg/h.

Then, the slurry was withdrawn from the CDC and then filtered using a belt filter. The cake obtained by the filtration was supplied continuously to a column rectification device (KCP, the column measuring 3 inches in diameter and about 1 m in height, available from Kureha Techno Eng K.K.), retained therein for one hour, and heated in the column top part with hot water of a temperature of 30°C - 50°C to produce refined NVP at a rate of 10.5 kg/h. The part of KCP and the agitation shafts were made of stainless steel, such as SUS 304, the feeder was made of SUS 304 coated with a fluororesin, and the gasket and the shaft seal were made of Teflon (a polytetrafluoroethylene resin). The refined NVP consequently obtained and 10 ppm of N,N'-di-sec-butyl-p-phenylenediamine added thereto as a stabilizer were put to storage together. Of the filtrate from the filter, the loss ratio of the raw material which was 3% of the virgin NVP, namely 0.3 kg/h, was discarded and the remainder was supplied, as mixed with the virgin NVP, to the CDC.

When the NVP obtained from the KCP was assayed at the time that the operation of the CDC was stabilized, it was found to have a purity of 99.9964%.

When the virgin NVP was made to add N, N' -di-sec-butyl-p-phenylenediamine as the stabilizer till the concentration thereof reached 0.25 ppm and was then treated in the same manner, the purity of the produced NVP was increased to 99.9980%. When the loss ratio of the raw material was decreased to the level equaled to the NVP purity obtained without adding the stabilizer, the NVP purity at the loss ratio of 1.5% was found to be 99.9965%. By addition of the stabilizer, the yield of rectification was improved by 1.5% (equivalent to 50% prior to the addition of the stabilizer).

Since the NVP obtained from the KCP was found to contain no detectable stabilizer therein, the refined NVP was obtained as a finished product after it had been made to add 10 ppm of N,N'-di-sec-butyl-p-phenylenediamine.

### EXAMPLE 2: CDC + KCP

An operation was performed by following the procedure of Example 1 while changing the amount of N,N'-di-sec-butyl-p-phenylenediamine added from 0.25 ppm to 1% by weight.

The NVP purity at the loss ratio of 1.5% was 99.9965%, indicating that the NVP was produced in a yield equivalent to the yield obtained when 0.25 ppm of the N,N'-di-sec-butyl-p-phenylenediamine was added.

### EXAMPLES 3 - 5: CDC + KCP

Operations were performed by following the procedure of Example 1 while using p-phenylenediamine, N,N-diethyl-p-phenylenediamine, and N,N'-diphenyl-p-phenylenediamine respectively in the same amount instead of N,N'-di-sec-butyl-p-phenylenediamine.

The NVP purities at the loss ratio of 1.5% were 99. 9960%, 99. 9962%, and 99. 9962% respectively, indicating that the NVPs were produced in yields substantially equivalent to the yield obtained when the N,N'-di-sec-butyl-p-phenylenediamine was used.

### EXAMPLE 6: CDC + KCP

An operation was performed by following the procedure of Example 1 while using 50 ppm of 2,6-di-t-butyl-4-hydroxy toluene instead of 0.25 ppm of N,N'-di-sec-butyl-p-phenylenediamine.

The NVP purity at the loss ratio of 1.5% was 99.9957%, indicating that the NVP was produced in a yield substantially equivalent to the yield obtained when 0.25 ppm of the N,N'-di-sec-butyl-p-phenylenediamine was added.

### EXAMPLE 7: Tank crystallization column + KCP

An operation was performed by following the procedure of Example 1 while using a tank crystallization column instead of CDC.

The NVP purity of 99.992% was obtained without the addition of N,N'-di-sec-butyl-p-phenylenediamine.

When N,N'-di-sec-butyl-p-phenylenediamine was added in the same concentration as in Example 1 to the virgin NVP, the purity of the produced NVP increased to 99.994%. When the loss ratio of the raw material was cut down till the NVP purity equaled to the purity obtained without addition of the N,N'-di-sec-butyl-p-phenylenediamine, a NVP purity of 99.992% was obtained at a loss ratio of 1.7%. By addition of the N,N'-di-sec-butyl-p-phenylenediamine, the yield of rectification was improved by 1.3% (corresponding to 43% of the yield obtained prior to addition of the N,N'-di-sec-butyl-p-phenylenediamine).

### EXAMPLE 8: Tank crystallization column

The raw material NVP (mother liquid) was continuously supplied to a conventional tank crystallization column to obtain crude crystals of NVP. The crystallization column was operated with the supplying speed of the mother liquid to the tank crystallization column set at 50 kg/h, including 8.25 kg/h of the virgin NVP, the concentration of the slurry set at 15%, the temperature set at 6.5°C, and the retention time at 2 hours.

Then, the slurry mentioned above was withdrawn from the tank crystallization column and filtered using a centrifugal separator. The cake resulting from the filtration was fused to obtain NVP at a ratio of 7.5 kg/h. Of the filtrate from the separator, the loss ratio of the raw material which was 10% of the virgin NVP, namely 0.75 kg/h, was discarded and the remainder was supplied again to the tank crystallization column.

When the NVP obtained from the centrifugal separator was assayed at the time that the operation in the tank crystallization column was stabilized, it was found to have a purity of 99.97%.

When the same operation was performed by adding N, N' -di-sec-butyl-p-phenylenediamine to the virgin NVP till the concentration thereof reached 0.5 ppm, the purity of the produced NVP increased to 99.98%. When the loss ratio of the raw material was decreased to a level equaled to the NVP purity obtained without the addition of N,N'-di-sec-butyl-p-phenylenediamine, the NVP purity was 99.97% at the loss ratio of 9.6%. By addition of the N,N'-di-sec-butyl-p-phenylenediamine, the yield of rectification was improved by 0.4% (corresponding to 4% of the yield obtained prior to the addition of N,N'-di-sec-butyl-p-phenylenediamine).

Since the NVP consequently obtained was found to contain no detectable N, N' -di-sec-butyl-p-phenylenediamine therein, it was obtained as a finished product after it had been made to add 10 ppm of N,N'-di-sec-butyl-p-phenylenediamine.

### EXAMPLE 9: CDC

An operation was performed by following the procedure of Example 8 while using a CDC instead of tank crystallization column. When the product was tested for confirming the yield by the addition of N, N'-di-sec-butyl-p-phenylenediamine, the improvement was found to be 0.4% (corresponding to 4% of the yield obtained prior to the addition of N,N'-di-sec-butyl-p-phenylenediamine). The results equaled to those obtained in Example 6.

### Industrial Applicability

This process can be applied for rectifying N-vinyl-2-pyrrolidone by crystallization.

## Claims

1. A process for rectifying N-vinyl-2-pyrrolidone by crystallization, **characterized in that** the crystallization is performed for a mother liquid having a crude N-vinyl-2-pyrrolidone in the presence of a stabilizer.

2. A process according to claim 1, wherein the stabilizer is a phenol oxidation inhibitor or an amine polymerization inhibitor.

3. A process according to claim 1 or claim 2, wherein an amount of the stabilizer is not more than 1.5 % by weight (not inclusive of 0) based on the weight of the crude N-vinyl-2-pyrrolidone.

4. A process according to claim 1, further comprising filtrating the resulting slurry obtained from the crystallization, and rectifying a crude crystal obtained from the filtration.

## Patentansprüche

1. Verfahren zur Rektifikation von N-Vinyl-2-pyrrolidon durch Kristallisation, **dadurch gekennzeichnet, dass** man die Kristallisation mit einer Mutterflüssigkeit mit rohem N-Vinyl-2-pyrrolidon in Gegenwart eines Stabilisators durchführt.

2. Verfahren nach Anspruch 1, wobei der Stabilisator ein phenolischer Oxidationsinhibitor oder ein aminischer Polymerisationsinhibitor ist.

3. Verfahren nach Anspruch 1 oder 2, wobei ein Anteil des Stabilisators nicht mehr als 1,5 Gewichts-% (nicht einschließlich 0), bezogen auf das Gewicht des rohen N-Vinyl-2-pyrrolidons, ist.

4. Verfahren nach Anspruch 1, bei dem man ferner die resultierende, aus der Kristallisation erhaltene Aufschlämmung filtriert, und einen Rohkristall, der aus der Filtration erhalten wird, rektifiziert.

## Revendications

1. Procédé pour la rectification du N-vinyl-2-pyrrolidone par cristallisation, cacarctérisé en ce que la cristallisation est exécuté pour un liquide-mère ayant un N-vinyl-2-pyrrolidone brut en présence d'un stabilisant

2. Procédé d'après la revendication 1, dans lequel le stabilisant est un inhibiteur de l'oxydation du phénol ou un inhibiteur de la polymérisation d'une amine.

3. Procédé d'après les revendications 1 ou 2, dans lequel une quantité du stabilisant n'est plus de 1,5% en poids (0 exclué) basée sur le poids du N-vinyl-2-pyrrolidone brut.

4. Procédé d'après la revendication 1, comportant de plus la filtration de la boue résultante obtenue de la cristallisation, et la rectification d'un cristal brut obtenu de la filtration.
